# EUROPÄISCHE PATENTSCHRIFT

(11) **EP 4 192 366 B1**
(45) Veröffentlichungstag und Bekanntmachung des Hinweises auf die Patenterteilung: **24.07.2024**
(21) Anmeldenummer: 21762636.5
(22) Anmeldetag: 05.08.2021
(51) Int. Cl.: A61B 17/16

(54) **CHIRURGISCHES INSTRUMENT**
SURGICAL INSTRUMENT
INSTRUMENT CHIRURGICAL

(30) Priorität: 07.08.2020 DE 102020120871
(43) Veröffentlichungstag der Anmeldung: 14.06.2023
(73) Patentinhaber: Efinger, Günter, 78549 Spaichingen (DE); Efinger, Levin, 78549 Spaichingen (DE)
(72) Erfinder: Efinger, Günter, 78549 Spaichingen (DE); Efinger, Levin, 78549 Spaichingen (DE)
(74) Vertreter: Westphal, Mussgnug & Partner Patentanwälte mbB
(86) Internationale Anmeldenummer: PCT/EP2021/071932
(87) Internationale Veröffentlichungsnummer: WO 2022/029261

(56) Entgegenhaltungen:
- DE-A1- 102013 010 973
- DE-A1- 19 602 511
- DE-A1- 19 702 079

## Beschreibung

Die Erfindung betrifft ein chirurgisches Instrument gemäß dem Oberbegriff des Patentanspruchs 1.

Bekannt sind chirurgische Instrument mit einem Basisteil umfassend einen Schaft und ein feststehendes Griffteil, wobei an dem Schaft ein Schiebeteil verschiebbar geführt angeordnet ist, und wobei an dem Basisteil zur Betätigung des Schiebeteils ein schwenkbares Griffteil mittels eines Gelenkbolzens schwenkbar angeordnet ist. Mittels des Schiebers wird ein Arbeitsende im distalen Endbereich des chirurgischen Instruments betätigt, welches beispielsweise als Stanze oder Klemme ausgebildet sein kann.

Um das chirurgische Instrument nach Gebrauch reinigen zu können, sind zerlegbare chirurgische Instrumente bekannt, bei welchen das Schiebeteil vollständig von dem Basisteil abgenommen werden kann. Dazu sind verschiedene Verriegelungsmechanismen bekannt, welche in einer ersten Position das Abnehmen des Schiebeteils unterbinden und in einer zweiten Position das Abnehmen des Schiebeteils erlauben. Ein Beispiel eines derartigen chirurgischen Instruments offenbart die de 299 22 271 U1.

Nachteilig bei derartigen chirurgischen Instrumenten ist, dass auf einem Reinigungstablett bei Reinigung mehrerer derartiger chirurgischer Instrumente die Schiebeteile nicht bei den Basisteilen verbleiben und es mühsam ist, die zueinander passenden Einzelteile nach der Reinigung zusammen zu suchen.

Daher sind ebenfalls bekannt chirurgische Instrumente, bei welchen das Schiebeteil in einer Reinigungsposition unverlierbar an dem Basisteil angeordnet ist. Die DE 199 21 614 A1 offenbart beispielsweise eine bewegbare Verbindung zwischen einem proximalen Ende des Schiebeteils und dem Basisteil, über welche das Schiebeteil in einer Reinigungsposition unverlierbar an dem Basisteil befestigt verbleibt, während sowohl die Verbindung zwischen dem Schiebeteil und dem schwenkbaren Griffteil als auch die Verbindung zwischen dem distalen Ende des Schiebeteils und dem Schaft gelöst wird.

Die EP 1 092 397 B1 offenbart ein chirurgisches Instrument, bei welchem das Schiebeteil nach Lösen eines Verriegelungsmechanismus weiter nach proximal verschoben werden kann, damit distal die T-Nuten außer Eingriff gebracht werden können, um das Schiebeteil distal vom Basisteil abheben und in eine Reinigungsposition verschwenken zu können, während es proximal an dem schwenkbaren Griffteil angelenkt verbleibt.

Die EP 2 941 207 B1, die EP 2 814 407 B1, die DE 20 2012 001348 U2, die DE 10 2009 056 099 B4 und die DE 10 2009 060 595 A1 offenbaren chirurgische Instrumente, bei welchen jeweils nach Lösen eines Verriegelungsmechanismus die schwenkbare Verbindung zwischen dem schwenkbaren Griffteil und dem Schiebeteil gelöst werden kann, so dass das Schiebeteil proximal vom Basisteil abgehoben werden kann, während es distal an dem Basisteil angelenkt verbleibt.

Das Dokument DE19702079 A1 offenbart ein chirurgisches Instrument gemäß dem Oberbegriff des Patentanspruchs 1.

Bei den bekannten chirurgischen Instrumenten ist das Schiebeteil in der Reinigungsposition über eine einzige Verbindung mit den weiteren Komponenten des Instruments verbunden. Dabei besteht bei der Reinigung die Gefahr, dass sich Instrumente ineinander verhaken und die Verbindung beschädigt wird.

Die Aufgabe der Erfindung besteht daher darin, ein chirurgisches Instrument bereitzustellen, bei welchem auch in einer Reinigungsposition die einzelnen Komponenten relativ zueinander stabil angeordnet sind.

Die Aufgabe wird erfindungsgemäß gelöst durch ein chirurgisches Instrument mit den Merkmalen des Patentanspruchs 1.

Vorteilhafte Ausgestaltungen und Weiterbildungen sind in den abhängigen Ansprüchen angegeben.

Das erfindungsgemäße chirurgisches Instrument mit einem Basisteil umfassend einen Schaft und ein feststehendes Griffteil, wobei an dem Schaft ein Schiebeteil verschiebbar geführt angeordnet ist, wobei an dem Basisteil zur Betätigung des Schiebeteils ein schwenkbares Griffteil mittels eines Gelenkbolzens schwenkbar angeordnet ist, wobei das Schiebeteil einen distalen Endbereich und einen proximalen Endbereich aufweist, wobei das Schiebeteil in dem proximalen Endbereich schwenkbar mit dem schwenkbaren Griffteil verbunden ist und das Schiebeteil in dem distalen Endbereich mit einer an dem Schaft angeordneten Betätigungsvorrichtung für ein Arbeitsende verbunden ist, zeichnet sich dadurch aus, dass das schwenkbare Griffteil unlösbar mit dem Schiebeteil und unlösbar mit der Betätigungsvorrichtung verbunden ist, dass das schwenkbare Griffteil ein Langloch aufweist, welches von dem Gelenkbolzen durchsetzt ist, und dass eine Verriegelungsvorrichtung vorgesehen ist, welche in einer ersten Position ein Verschieben des Gelenkbolzens entlang des Langlochs unterbindet und welche in einer zweiten Position ein Verschieben des Gelenkbolzens entlang des Langlochs erlaubt.

Der Erfindung liegt die Idee zugrunde, weder die Verbindung zwischen dem Schiebeteil und der Betätigungsvorrichtung für das Arbeitsende noch die Verbindung zwischen dem Schiebeteil und dem schwenkbaren Griffteil zu lösen, sondern das schwenkbare Griffteil verschiebbar an dem Basisteil anzuordnen, um dadurch ein Abheben der Schiebeteils von dem Basisteil ermöglichen zu können. Sowohl in der Arbeitsposition, in welcher das Schiebeteil verschiebbar an dem Schaft des Basisteils angeordnet ist, als auch in der Reinigungsposition, in welcher das Schiebeteil von dem Schaft abgehoben, insbesondere im proximalen Endbereich abgehoben ist, bleibt das Schiebeteil sowohl mit dem Schaft als auch mit dem schwenkbaren Griffteil verbunden, wodurch auch in der Reinigungsposition eine stabile Anordnung der Komponenten zueinander erreicht werden kann.

Eine bevorzugte Ausführungsform der Erfindung sieht vor, dass das schwenkbare Griffteil einen Durchbruch des Basisteils durchsetzt. Dadurch kann ein besonders kompakter Aufbau erreicht werden.

Gemäß einer besonders bevorzugten Weiterbildung der Erfindung umfasst die Verriegelungsvorrichtung den Gelenkbolzen, welcher in Richtung seiner Längsachse zwischen einer ersten Position und einer zweiten Position verschiebbar ist. Eine derartige Ausgestaltung kann die Zahl der erforderlichen Komponenten verringern, da dem Gelenkbolzen eine Doppelfunktion zugewiesen werden kann.

Vorteilhafterweise weist der Gelenkbolzen einen ersten Abschnitt mit einem ersten Durchmesser und einen zweiten Abschnitt mit einem zweiten Durchmesser, wobei der erste Durchmesser größer ist als der zweite Durchmesser, und das Langloch einen ersten Abschnitt mit einem ersten Durchmesser und einen zweiten Abschnitt mit einem zweiten Durchmesser auf, wobei der erste Durchmesser größer ist als der zweite Durchmesser, wobei in der ersten Position der Verriegelungsvorrichtung der erste Abschnitt des Gelenkbolzens das Langloch, insbesondere den ersten Abschnitt des Langlochs, und in der zweiten Position der Verriegelungsvorrichtung der zweite Abschnitt des Gelenkbolzens das Langloch durchsetzt. Mit einer derartigen Ausgestaltung kann auf einfache und kompakte Art und Weise eine Verriegelungsvorrichtung realisiert werden.

Eine vorteilhafte Ausgestaltung der Erfindung sieht vor, dass das Langloch einen dritten Abschnitt mit einem dritten Durchmesser aufweist, wobei der zweite Abschnitt zwischen dem ersten Abschnitt und dem dritten Abschnitt angeordnet ist, und wobei der dritte Durchmesser größer als der zweite Durchmesser ist und insbesondere dem ersten Durchmesser entspricht. Eine derartige Ausgestaltung des Langlochs ermöglicht ein Verriegeln des schwenkbaren Griffteils und somit des Schiebeteils in der Reinigungsposition, so dass ein unbeabsichtigtes Überführen des Schiebeteils in die Arbeitsposition während des Reinigungsprozesses vermieden werden kann.

Besonders bevorzugt ist der Gelenkbolzen durch ein Federelement beaufschlagt, insbesondere derart, dass der Gelenkbolzen durch das Federelement beaufschlagt in der ersten Position angeordnet ist und gegen die Kraft des Federelements aus der ersten Position in die zweite Position überführbar ist. Dadurch kann gewährleistet werden, dass die Verriegelungsvorrichtung nicht unbeabsichtigt in eine Entriegelungsposition überführt wird.

Vorzugsweise ist das schwenkbare Griffteil über einen Achsstift unlösbar mit dem Schiebeteil verbunden, wodurch eine einfache schwenkbare, unlösbare Verbindung ermöglicht werden kann.

Vorteilhafterweise ist das schwenkbare Griffteil über einen Achsstift unlösbar mit der Betätigungsvorrichtung verbunden, wodurch eine einfache schwenkbare, unlösbare Verbindung ermöglicht werden kann.

Eine besonders bevorzugte Weiterbildung der Erfindung sieht vor, dass an dem Schiebeteil ein Führungsvorsprung und an dem Basisteil eine entsprechende Führungsnut angeordnet sind. Dadurch kann die Stabilität der Bewegung des Schiebeteils an dem Schaft erhöht werden.

Ein Ausführungsbeispiel der Erfindung wird anhand der folgenden Figuren ausführlich erläutert. Es zeigen
- Figur 1: eine perspektivische Ansicht eines Ausführungsbeispiels eines erfindungsgemäßen chirurgischen Instruments in einer Reinigungsposition,
- Figur 2a: eine Seitenansicht des chirurgischen Instruments gemäß Figur 1 in einer Arbeitsposition,
- Figur 2b: einen Schnitt durch das chirurgische Instrument entlang der Linie A-A in Figur 2a,
- Figur 3a: eine Seitenansicht des chirurgischen Instruments gemäß Figur 1 in der Reinigungsposition,
- Figur 3b: einen Schnitt durch das chirurgische Instrument entlang der Linie B-B in Figur 3a,
- Figur 4a: eine perspektivische Ansicht eines ersten Teils eines Gelenkbolzens des chirurgischen Instruments gemäß Figur 1,
- Figur 4b: eine Seitenansicht des ersten Teils des Gelenkbolzens gemäß Figur 4a,
- Figur 4c: eine perspektivische Ansicht eines zweiten Teile des Gelenkbolzens des chirurgischen Instruments gemäß Figur 1,
- Figur 4d: eine Seitenansicht des zweiten Teils des Gelenkbolzens gemäß Figur 4c,
- Figur 4e: einen Schnitt durch das zweite Teil des Gelenkbolzens entlang der Linie B-B in Figur 4d und
- Figur 5: eine Seitenansicht des schwenkbaren Griffteils des chirurgischen Instruments gemäß Figur 1.

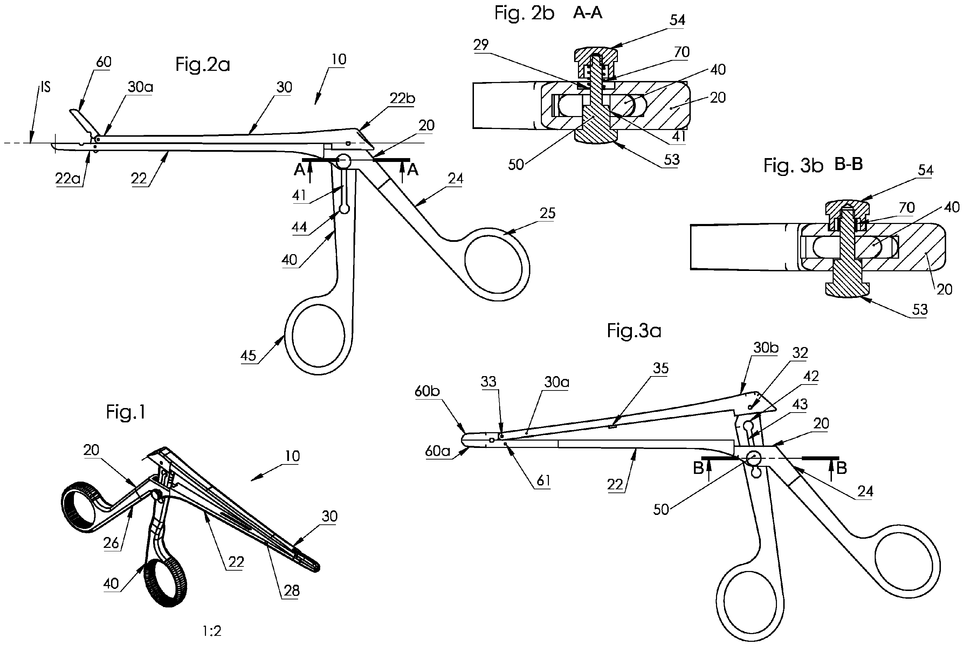

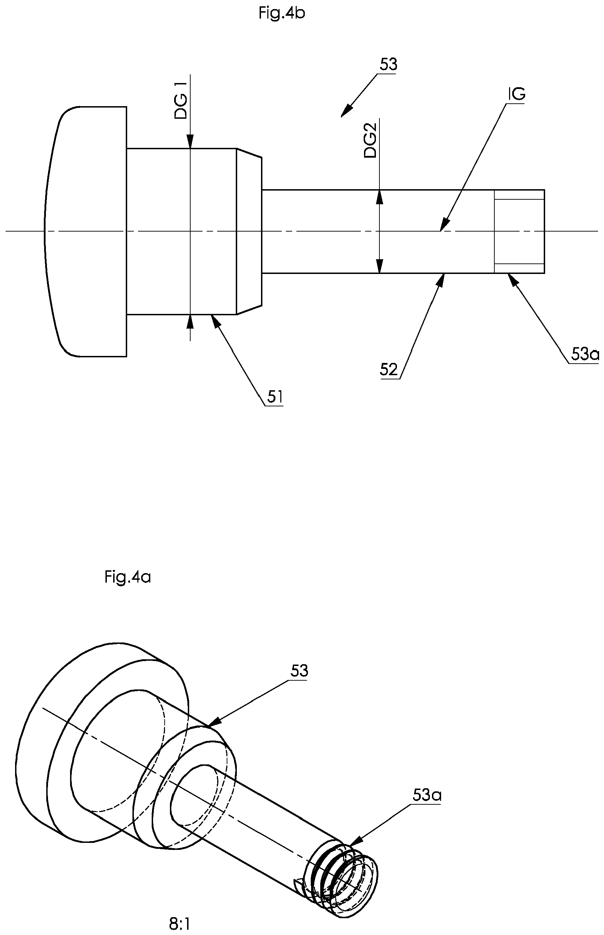

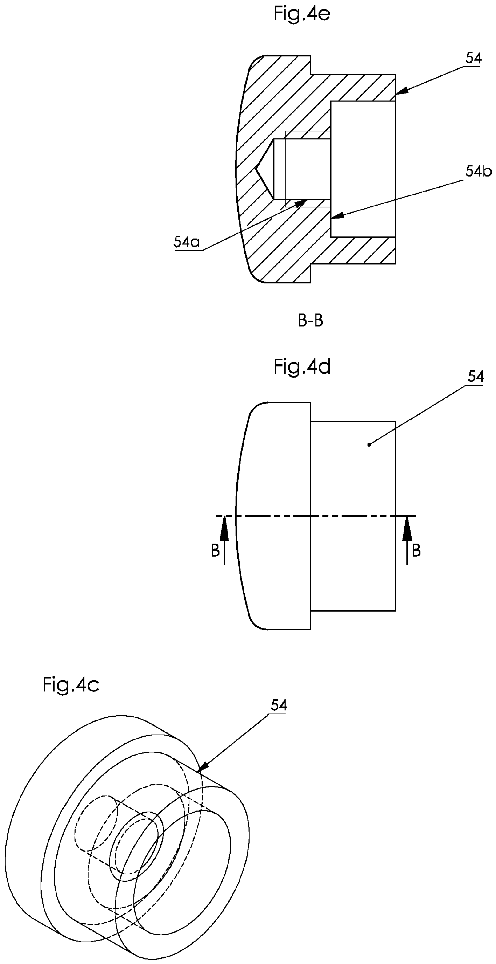

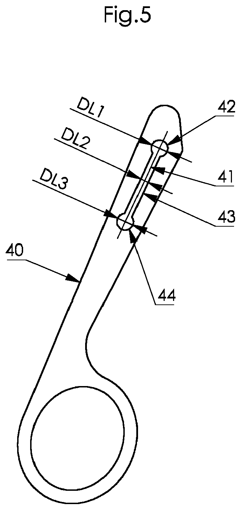

Die Figuren 1, 2a, 2b, 3a und 3b zeigen verschiedene Ansichten eines Ausführungsbeispiels eines erfindungsgemäßen chirurgischen Instruments 10, die Figuren 4a bis 4e und 5 zeigen Ansichten von Komponenten des chirurgischen Instruments 10 gemäß Figur 1. Zur besseren Übersicht sind nicht sämtliche Bezugsziffern in sämtlichen Figuren angegeben.

Das chirurgische Instrument 10 weist ein Basisteil 20 umfassend einen Schaft 22, welcher einen distalen Endbereich 22a und einen proximalen Endbereich 22b aufweist, und ein feststehendes Griffteil 24 auf. Der Schaft 22 weist eine Längsachse lS auf, wobei das Griffteil 24 insbesondere gegen die Längsachse lS abgewinkelt an dem proximalen Endbereich 22b des Schafts 22 angeordnet sein kann.

An dem Schaft 22 ist ein Schiebeteil 30, welches einen distalen Endbereich 30a und einen proximalen Endbereich 30b aufweist, verschiebbar geführt angeordnet. Dabei ist das Schiebeteil 30 im Wesentlichen parallel zu der Längsachse lS verschiebbar an dem Schaft 22 geführt. An dem Basisteil 20 ist zur Betätigung des Schiebeteils 30 ein schwenkbares Griffteil 40 mittels eines Gelenkbolzens 50, welcher eine Längsachse lG aufweist, schwenkbar angeordnet. Das feststehende Griffteil 24 und das schwenkbare Griffteil 40 können jeweils eine Grifföse 25, 45 aufweisen, um die Handhabung zu erleichtern.

In dem Basisteil 20 kann ein Durchbruch 26 angeordnet sein, welcher von dem schwenkbaren Griffteil 40 durchsetzt wird. Weiterhin kann der Gelenkbolzen 50 in dem Durchbruch das schwenkbare Griffteil 40 durchsetzen und in den Seitenwänden des Durchbruchs an dem Basisteil 20 gelagert angeordnet sein.

Um die Schiebebewegung geführt vornehmen zu können, kann an dem Schiebeteil 30 ein Führungsvorsprung 35 und an dem Basisteil, insbesondere an dem Schaft 22, eine entsprechende Führungsnut 28, in welche der Führungsvorsprung 35 in der Arbeitsposition eingreifen kann, angeordnet sein.

Das Schiebeteil 30 ist in seinem proximalen Endbereich 30b schwenkbar und unlösbar mit dem schwenkbaren Griffteil 40 verbunden. Dazu ist insbesondere ein Achsstift 32 vorgesehen, welcher eine Durchgangsöffnung in dem schwenkbaren Griffteil 40 durchsetzt und an seinen freien Enden in in dem Schiebeteils 30 angeordneten Öffnungen angeordnet und dort beispielsweise verpresst sein kann. Weiterhin ist das Schiebeteil 30 in seinem distalen Endbereich 30a mit einer an dem Schaft 22 angeordneten Betätigungsvorrichtung 61 für ein Arbeitsende 60 verbunden.

Das Arbeitsende 60 kann beispielsweise als Klemme oder als Schere ausgebildet sein und zwei gegeneinander verschwenkbare Maulteile 60a, 60b aufweisen. Dabei kann das Maulteil 60a durch den distalen Endbereich 22a des Schafts 22 ausgebildet und damit insbesondere feststehend ausgebildet sein. Das Maulteil 60b kann über eine Betätigungsvorrichtung 60 schwenkbar an dem Schaft 22 angeordnet sein, wobei über die Betätigungsvorrichtung 60 die Längsbewegung des Schiebeteils 30 parallel zur Längsachse lS des Schafts in eine Schwenkbewegung des Maulteils 60b umgesetzt wird.

Die Verbindung zwischen dem Schiebeteil 30 und der Betätigungsvorrichtung 61 ist unlösbar. Dazu kann ein Achsstift 33 vorgesehen sein, welcher eine Durchgangsöffnung beispielsweise in einem Hebelelement der Betätigungsvorrichtung 61 durchsetzt und an seinen freien Enden in in dem Schiebeteil 30 angeordneten Öffnungen angeordnet und dort beispielsweise verpresst sein kann.

Das schwenkbare Griffteil 40 weist ein Langloch 41 auf, welches von dem Gelenkbolzen 50 durchsetzt ist. Das Langloch 41 ermöglicht es, das schwenkbare Griffteil 40 zu verschieben, insbesondere zwischen der Arbeitsposition, in welcher das Schiebeteil 30 verschiebbar an dem Schaft 22 des Basisteils 20 angeordnet ist (vgl. Figur 2a), und der Reinigungsposition, in welcher das Schiebeteil 30 von dem Schaft 22 abgehoben ist (vgl. Figuren 1 und 3a). Insbesondere kann das schwenkbare Griffteil 40 dazu durch den Durchbruch 26 des Basisteils geschoben werden.

Es ist eine Verriegelungsvorrichtung vorgesehen ist, welche in einer ersten Position ein Verschieben des Gelenkbolzens 50 entlang des Langlochs 41 oder mit anderen Worten ein Verschieben des schwenkbaren Griffteils 40 gegen das Basisteil 20 unterbindet und welche in einer zweiten Position ein Verschieben des Gelenkbolzens 50 entlang des Langlochs 41 oder mit anderen Worten ein Verschieben des schwenkbaren Griffteils 40 gegen das Basisteil 20 erlaubt.

Die Verriegelungsvorrichtung umfasst gemäß der dargestellten Ausführungsform den Gelenkbolzen 50, welcher in Richtung seiner Längsachse lG zwischen einer ersten Position (vgl. Figur 2b) und einer zweiten Position (vgl. Figur 3b) verschiebbar ist.

Der Gelenkbolzen 50 kann einen ersten Abschnitt 51 mit einem ersten Durchmesser DG1 und einen zweiten Abschnitt 52 mit einem zweiten Durchmesser DG2 aufweist, wobei der erste Durchmesser DG1 größer ist als der zweite Durchmesser DG2. Weiterhin kann das Langloch 41 einen ersten Abschnitt 42 mit einem ersten Durchmesser DL1 und einen zweiten Abschnitt 43 mit einem zweiten Durchmesser DL2 aufweisen, wobei der erste Durchmesser DL1 größer ist als der zweite Durchmesser DL2. In der ersten Position der Verriegelungsvorrichtung durchsetzt der erste Abschnitt 51 des Gelenkbolzens 50 das Langloch 41, insbesondere den ersten Abschnitt 42 des Langlochs 41, und in der zweiten Position der Verriegelungsvorrichtung durchsetzt der zweite Abschnitt 52 des Gelenkbolzens 50 das Langloch 41. Die Durchmesser DL1, DL2, DG1, DG2 sind insbesondere derart aufeinander abgestimmt, dass in der ersten Position der erste Abschnitt 51 des Gelenkbolzens 50 nicht in den zweiten Abschnitt 43 des Langlochs 41 gleiten kann, da der erste Durchmesser DG1 des ersten Abschnitts 51 des Gelenkbolzens 50 größer ist als der zweite Durchmesser DL2 des zweiten Abschnitts 43 des Langlochs 41. Dadurch kann das schwenkbare Griffteil 40 in der Arbeitsposition, in welcher die Verriegelungsvorrichtung und insbesondere der Gelenkbolzen 50 in der ersten Position angeordnet ist, arretiert werden (vgl. Figur 2a). Erst bei Überführen der Verriegelungsvorrichtung und insbesondere des Gelenkbolzens 50 in die zweite Position wird der zweite Abschnitt 52 des Gelenkbolzens 50 in das Langloch 41 überführt, wobei der zweite Durchmesser DG2 des zweiten Abschnitts 52 des Gelenkbolzens geringfügig kleiner ist als der zweite Durchmesser DL2 des zweiten Abschnitts 43 des Langlochs 41, so dass der zweite Abschnitt 52 des Gelenkbolzens entlang des Langlochs 41 gleitend geführt verschoben werden kann. Dabei wird insbesondere das schwenkbare Griffteil 40 durch den Durchbruch 26 des Basisteils 20 derart verschoben, dass der proximale Endbereich 22b des Schiebeteils 20 von dem proximalen Endbereich 22b des Schafts 22 abgehoben wird und in eine Reinigungsposition überführt wird (vgl. Figuren 1 und 3a).

Das Langloch 41 kann einen dritten Abschnitt 44 mit einem dritten Durchmesser DL3 aufweisen, wobei der zweite Abschnitt 43 zwischen dem ersten Abschnitt 42 und dem dritten Abschnitt 44 angeordnet ist, und wobei der dritte Durchmesser DL3 größer als der zweite Durchmesser DL2 ist und insbesondere dem ersten Durchmesser DL1 entspricht. Dies ermöglicht es, dass der Gelenkbolzen 50 in dem Fall, dass er den dritten Abschnitt 44 des Langlochs 41 durchsetzt, wieder aus der zweiten Position in die erste Position überführt werden kann, in welcher der erste Abschnitt 51 des Gelenkbolzens 50 das Langloch 41 durchsetzt, so dass eine Verriegelung in der Reinigungsposition ermöglicht werden kann.

Der Gelenkbolzen 50 kann zweiteilig ausgebildet sein und ein erstes Teil 53 und ein zweites Teil 54 umfassen, welches beispielsweise mittels eines Gewindes miteinander verbunden sein können. Beispielsweise kann das erste Teil 53 ein Außengewinde 53a und das zweite Teil 54 ein Innengewinde 54a aufweisen. Das erste Teil 53 kann den ersten Abschnitt 51 und den zweiten Abschnitt 52 umfassen.

Der Gelenkbolzen 50 kann durch ein Federelement 70 beaufschlagt sein, insbesondere derart, dass der Gelenkbolzen 50 durch das Federelement 70 beaufschlagt in der ersten Position angeordnet ist und gegen die Kraft des Federelements 70 aus der ersten Position in die zweite Position überführbar ist. Das Federelement 70 kann beispielsweise als Schraubenfeder ausgebildet sein und sich einenends an einer Anlagefläche 54b des Gelenkbolzens 50 und anderenends an einer Anlagefläche 29 des Basisteils 20 abstützen. Durch das Federelement 70 kann sichergestellt werden, dass keine unbeabsichtigte Entriegelung erfolgt.

### Bezugszeichenliste

- 10: chirurgisches Instrument
- 20: Basisteil
- 22: Schaft
- 22a: distaler Endbereich
- 22b: proximaler Endbereich
- 24: feststehendes Griffteil
- 25: Grifföse
- 26: Durchbruch
- 28: Führungsnut
- 29: Anlagefläche
- 30: Schiebeteil
- 30a: distaler Endbereich
- 30b: proximaler Endbereich
- 32: Achsstift
- 33: Achsstift
- 35: Führungsvorsprung
- 40: schwenkbares Griffteil
- 41: Langloch
- 42: erster Abschnitt
- 43: zweiter Abschnitt
- 44: dritter Abschnitt
- 45: Grifföse
- 50: Gelenkbolzen
- 51: erster Abschnitt
- 52: zweiter Abschnitt
- 53: erstes Teil
- 53a: Außengewinde
- 54: zweites Teil
- 54a: Innengewinde
- 54b: Anlagefläche
- 60: Arbeitsende
- 60a: Maulteil
- 60b: Maulteil
- 61: Betätigungsvorrichtung
- 70: Federelement
- lG: Längsachse des Gelenkbolzens
- lS: Längsachse des Schafts
- DG1: erster Durchmesser
- DG2: zweiter Durchmesser
- DL1: erster Durchmesser
- DL2: zweiter Durchmesser
- DL3: dritter Durchmesser

## Patentansprüche

1. Chirurgisches Instrument (10) mit einem Basisteil (20) umfassend einen Schaft (22) und ein feststehendes Griffteil (24), wobei an dem Schaft (22) ein Schiebeteil (30) verschiebbar geführt angeordnet ist, wobei an dem Basisteil (20) zur Betätigung des Schiebeteils (30) ein schwenkbares Griffteil (40) mittels eines Gelenkbolzens (50) schwenkbar angeordnet ist, wobei das Schiebeteil (30) einen distalen Endbereich (30a) und einen proximalen Endbereich (30b) aufweist, wobei das Schiebeteil (30) in dem proximalen Endbereich (30b) schwenkbar mit dem schwenkbaren Griffteil (40) verbunden ist und das Schiebeteil (30) in dem distalen Endbereich (30a) mit einer an dem Schaft (22) angeordneten Betätigungsvorrichtung (61) für ein Arbeitsende (60) verbunden ist,
wobei das schwenkbare Griffteil (40) ein Langloch (41) aufweist, welches von dem Gelenkbolzen durchsetzt (50) ist, wobei
eine Verriegelungsvorrichtung vorgesehen ist, welche in einer ersten Position ein Verschieben des Gelenkbolzens (50) entlang des Langlochs (41) unterbindet und welche in einer zweiten Position ein Verschieben des Gelenkbolzens (50) entlang des Langlochs (41) erlaubt,
**dadurch gekennzeichnet, dass** das schwenkbare Griffteil (40) unlösbar mit dem Schiebeteil (30) und unlösbar mit der Betätigungsvorrichtung (61) verbunden ist.

2. Chirurgisches Instrument nach Anspruch 1,
**dadurch gekennzeichnet, dass** das schwenkbare Griffteil (40) einen Durchbruch (26) des Basisteils (20) durchsetzt.

3. Chirurgisches Instrument nach einem der vorherstehenden Ansprüche,
**dadurch gekennzeichnet, dass** die Verriegelungsvorrichtung den Gelenkbolzen (50) umfasst, welcher in Richtung seiner Längsachse (lG) zwischen einer ersten Position und einer zweiten Position verschiebbar ist.

4. Chirurgisches Instrument nach einem der vorherstehenden Ansprüche,
**dadurch gekennzeichnet, dass** der Gelenkbolzen (50) einen ersten Abschnitt (51) mit einem ersten Durchmesser (DG1) und einen zweiten Abschnitt (52) mit einem zweiten Durchmesser (DG2) aufweist, wobei der erste Durchmesser (DG1) größer ist als der zweite Durchmesser (DG2), und dass das Langloch (41) einen ersten Abschnitt (42) mit einem ersten Durchmesser (DL1) und einen zweiten Abschnitt (43) mit einem zweiten Durchmesser (DL2) aufweist, wobei der erste Durchmesser (DL1) größer ist als der zweite Durchmesser (DL2), und dass in der ersten Position der Verriegelungsvorrichtung der erste Abschnitt (51) des Gelenkbolzens (50) das Langloch (41), insbesondere den ersten Abschnitt (42) des Langlochs (41), durchsetzt und in der zweiten Position der Verriegelungsvorrichtung der zweite Abschnitt (52) des Gelenkbolzens (50) das Langloch (41) durchsetzt.

5. Chirurgisches Instrument nach einem der vorherstehenden Ansprüche,
**dadurch gekennzeichnet, dass** das Langloch (41) einen dritten Abschnitt (44) mit einem dritten Durchmesser (DL3) aufweist, wobei der zweite Abschnitt (43) zwischen dem ersten Abschnitt (42) und dem dritten Abschnitt (44) angeordnet ist, und wobei der dritte Durchmesser (DL3) größer als der zweite Durchmesser (DL2) ist und insbesondere dem ersten Durchmesser (DL1) entspricht.

6. Chirurgisches Instrument nach einem der vorherstehenden Ansprüche,
**dadurch gekennzeichnet, dass** der Gelenkbolzen (50) durch ein Federelement (70) beaufschlagt ist, insbesondere derart, dass der Gelenkbolzen (50) durch das Federelement (70) beaufschlagt in der ersten Position angeordnet ist und gegen die Kraft des Federelements (70) aus der ersten Position in die zweite Position überführbar ist.

7. Chirurgisches Instrument nach einem der vorherstehenden Ansprüche,
**dadurch gekennzeichnet, dass** das schwenkbare Griffteil (40) über einen Achsstift (32) unlösbar mit dem Schiebeteil (30) verbunden ist.

8. Chirurgisches Instrument nach einem der vorherstehenden Ansprüche,
**dadurch gekennzeichnet, dass** das schwenkbare Griffteil (40) über einen Achsstift (33) unlösbar mit der Betätigungsvorrichtung (61) verbunden ist.

9. Chirurgisches Instrument nach einem der vorherstehenden Ansprüche,
**dadurch gekennzeichnet, dass** an dem Schiebeteil (30) ein Führungsvorsprung (35) und an dem Basisteil (20) eine entsprechende Führungsnut (28) angeordnet sind.

## Claims

1. Surgical instrument (10) having a base part (20) which includes a shaft (22) and a stationary handle part (24), wherein a sliding part (30) is arranged displaceably guided on the shaft (22), wherein a pivotable handle part (40) for actuating the sliding part (30) is pivotably arranged on the base part (20) by means of a pivot pin (50), wherein the sliding part (30) comprises a distal end region (30a) and a proximal end region (30b), wherein the sliding part (30) is pivotably connected to the pivotable handle part (40) in the proximal end region (30b) and the sliding part (30) is connected in the distal end region (30a) to an actuating mechanism (61) for an operating end (60) arranged on the shaft (22), wherein the pivotable handle part (40) comprises an elongated hole (41) which is penetrated by the pivot pin (50), wherein a locking mechanism is provided which, in a first position, prevents a sliding of the pivot pin (50) along the elongated hole (41) and which, in a second position, allows a sliding of the pivot pin (50) along the elongated hole (41),
**characterized in that** the pivotable handle part (40) is non-detachably connected to the sliding part (30) and non-detachably connected to the actuating mechanism (61).

2. Surgical instrument in accordance with claim 1,
**characterized in that** the pivotable handle part (40) penetrates a through hole (26) of the base part (20).

3. Surgical instrument in accordance with either of the preceding claims,
**characterized in that** the locking mechanism includes a pivot pin (50) which is displaceable in the direction of its longitudinal axis (1G) between a first position and a second position.

4. Surgical instrument in accordance with any of the preceding claims,
**characterized in that** the pivot pin (50) comprises a first section (51) with a first diameter (DG1) and a second section (52) with a second diameter (DG2), wherein the first diameter (DG1) is larger than the second diameter (DG2), and **in that** the elongated hole (41) comprises a first section (42) with a first diameter (DL1) and a second section (43) with a second diameter (DL2), wherein the first diameter (DL1) is larger than the second diameter (DL2), and **in that**, in the first position of the locking mechanism, the first section (51) of the pivot pin (50) penetrates the elongated hole (41), in particular the first section (42) of the elongated hole (41) and, in the second position of the locking mechanism, the second section (52) of the pivot pin (50) penetrates the elongated hole (41).

5. Surgical instrument in accordance with any of the preceding claims,
**characterized in that** the elongated hole (41) comprises a third section (44) with a third diameter (DL3), wherein the second section (43) is arranged between the first section (42) and the third section (44), and wherein the third diameter (DL3) is larger than the second diameter (DL2) and, in particular, corresponds to the first diameter (DL1).

6. Surgical instrument in accordance with any of the preceding claims,
**characterized in that** the pivot pin (50) is biased by means of a spring element (70), in particular such that the pivot pin (50), biased by means of the spring element (70), is arranged in the first position and can be moved against the force of the spring element (70) from the first position into the second position.

7. Surgical instrument in accordance with any of the preceding claims,
**characterized in that** the pivotable handle part (40) is non-detachably connected to the sliding part (30) by means of an axle pin (32).

8. Surgical instrument in accordance with any of the preceding claims,
**characterized in that** the pivotable handle part (40) is non-detachably connected to the actuating mechanism (61) by means of an axle pin (32).

9. Surgical instrument in accordance with any of the preceding claims,
**characterized in that** a guide projection (35) is arranged on the sliding part (30), and on the base part (20) a corresponding guide groove (28) is arranged.

## Revendications

1. Instrument chirurgical (10) comportant une pièce de base (20) avec une tige (22) et une poignée fixe (24),
- la tige (22) guidant de façon coulissante une pièce coulissante (30),
- la pièce de base (20) comportant une poignée pivotante (40) pour actionner la pièce coulissante (30), la poignée (40) étant montée pivotante sur un goujon d'articulation (50),
- la pièce coulissante (30) ayant une zone d'extrémité distale (30a) et une zone d'extrémité proximale (30b),
- la pièce coulissante (30) étant reliée de manière pivotante à la poignée pivotante (40) dans la zone d'extrémité proximale (30b) et la pièce coulissante (30) étant reliée dans la zone d'extrémité distale (30a) à un dispositif d'actionnement (61) installé sur la tige (22) pour une extrémité active (60),
- la poignée pivotante (40) comporte un trou oblong (41) traversé par le goujon d'articulation (50),
- un dispositif de verrouillage qui, dans une première position, interdit le coulissement du goujon d'articulation (50) le long du trou oblong (41) et qui, dans une seconde position, permet le coulissement du goujon d'articulation (50) le long du trou oblong (41),
instrument **caractérisé en ce que**
la poignée pivotante (40) est reliée de manière non détachable à la pièce coulissante (30) et de manière non détachable au dispositif d'actionnement (61).

2. Instrument chirurgical selon la revendication 1,
**caractérisé en ce que**
la poignée pivotante (40) traverse un passage (26) de la pièce de base (20).

3. Instrument chirurgical selon l'une des revendications précédentes,
**caractérisé en ce que**
le dispositif de verrouillage comprend le goujon d'articulation (50) coulissant selon son axe longitudinal (1G) entre une première position et une seconde position.

4. Instrument chirurgical selon l'une des revendications précédentes, **caractérisé en ce que**
- le goujon d'articulation (50) a un premier segment (51) ayant un premier diamètre (DG1) et un second segment (52) ayant un second diamètre (DG2),
* le premier diamètre (DG1) étant supérieur au second diamètre (DG2), et
- le trou oblong (41) a un premier segment (42) avec un premier diamètre (DL1) et un second segment (43) avec un second diamètre (DL2),
* le premier diamètre (DL1) étant supérieur au second diamètre (DL2), et
- dans la première position du dispositif de verrouillage le premier segment (51) du goujon d'articulation (50) traverse le trou oblong (41) notamment le premier segment (42) du trou oblong (41) et dans la seconde position du dispositif de verrouillage le second segment (52) du goujon d'articulation (50) traverse le trou oblong (41).

5. Instrument chirurgical selon l'une des revendications précédentes,
**caractérisé en ce que**
le trou oblong (41) a un troisième segment (44) avec un troisième diamètre (DL3),
- le second segment (43) étant situé entre le premier segment (42) et le troisième segment (44), et
- le troisième diamètre (DL3) est supérieur au second diamètre (DL2) et correspond notamment au premier diamètre (DL1).

6. Instrument chirurgical selon l'une des revendications précédentes,
**caractérisé en ce que**
le goujon d'articulation (50) est sollicité par un élément de ressort (70) notamment de façon que le goujon d'articulation (50) soit sollicité par l'élément de ressort (70) dans sa première position et puisse passer de sa première position dans la seconde position contre la force développée par l'élément de ressort (70).

7. Instrument chirurgical selon l'une des revendications précédentes, **caractérisé en ce que**
la poignée pivotante (40) est reliée par un axe (32) à la pièce coulissante (30).

8. Instrument chirurgical selon l'une des revendications précédentes,
**caractérisé en ce que**
la poignée pivotante (40) est reliée de manière non détachable par un axe (33) au dispositif d'actionnement (61).

9. Instrument chirurgical selon l'une des revendications précédentes,
**caractérisé en ce que**
la pièce coulissante (30) comporte un relief de guidage (35) et la pièce de base (20) a une rainure de guidage (28) correspondante.
